(19) Europäisches Patentamt / European Patent Office / Office européen des brevets

(11) **EP 2 236 124 A1**

(12) **EUROPEAN PATENT APPLICATION**
published in accordance with Art. 153(4) EPC

(43) Date of publication:
**06.10.2010 Bulletin 2010/40**

(51) Int Cl.:
*A61K 8/25* (2006.01)  *A45D 34/04* (2006.01)
*A61K 8/06* (2006.01)  *A61K 8/73* (2006.01)
*A61Q 1/14* (2006.01)

(21) Application number: **08870366.5**

(22) Date of filing: **10.12.2008**

(86) International application number:
**PCT/JP2008/072417**

(87) International publication number:
**WO 2009/087849 (16.07.2009 Gazette 2009/29)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **07.01.2008 JP 2008000616**

(71) Applicant: **Shiseido Company, Ltd.**
**Chuo-ku**
**Tokyo 104-8010 (JP)**

(72) Inventors:
• **IDE, Nobuyuki**
**Yokohama-shi**
**Kanagawa 224-8558 (JP)**
• **TAKATA, Motoki**
**Yokohama-shi**
**Kanagawa 224-8558 (JP)**

(74) Representative: **Green, Mark Charles**
**Urquhart-Dykes & Lord LLP**
**30 Welbeck Street**
**London W1G 8ER (GB)**

(54) **MAKEUP REMOVER AND DEVICE FOR MAKEUP REMOVAL**

(57) There is provided a cosmetic material removing agent including an expansive clay mineral and a dextrin fatty acid ester. There is provided a cosmetic material removing device including the cosmetic material removing agent as described above and a container for containing and feeding the cosmetic material removing agent.

FIG.1

**Description**

TECHNICAL FIELD

The present invention relates to a cosmetic material removing agent and a cosmetic material removing device.

BACKGROUND ART

Conventionally, a cleansing agent such as a cleansing cream or a cleansing oil is contained in a tissue or a cotton for use in wiping off an eye makeup for conducting removal thereof. However, it has become difficult to effectively remove cosmetic materials with different formulations such as mascaras, eyebrows, eye shadows and eye liners at once, along with the development of a cosmetic material in recent years.
In particular, with respect to mascaras, a product having a higher water resistance and being difficult to smudge has been developed and a fiber-containing mascara, a mascara foundation, and the like, for making an eyelash(es) look long have actively been developed. In order to remove these with a conventional cleansing agent, it has been necessary to contain and use a large amount of a cleansing agent in a tissue or a cotton whereby the amount of a cleansing agent for each use is large, which is uneconomical. Furthermore, rubbing of an eyelash(es) with a cotton containing a large amount of liquid many times involves a risk of causing a cleansing agent to enter an eye, causing damage on a comparatively thin and delicate skin surrounding an eye, or the like. Moreover, in a conventional cleansing method, it has been difficult to selectively remove only an eyelash cosmetic material such as a mascara, among eye makeup agents having already been applied.
In recent years, a type of mascara removing equipment and mascara removing agent has been developed in which a mascara remover contained in a container with an application tool is applied by using the application tool whereby it is unnecessary to contain and use a cleansing agent in a tissue or a cotton and it is considered that it is possible to reduce the amount of a used mascara remover (for example, see Japanese Patent Application Publication No. 2003-104839). However, such a mascara remover has some drawbacks whereby, for example, its adhesion to an eyelash(es) is not uniform due to its less-smooth liquid and its adhesion to an eyelash(es) is not constant due to a temperature dependence of the liquid, thus, the effect of removing a mascara is insufficient.

DISCLOSURE OF THE INVENTION

MEANS FOR SOLVING THE PROBLEM

According to one aspect of the present invention, there is provided a cosmetic material removing agent including an expansive clay mineral and a dextrin fatty acid ester.
According to another aspect of the present invention, there is provided a cosmetic material removing device including the cosmetic material removing agent as described above and a container for containing and feeding the cosmetic material removing agent.

BRIEF DESCRIPTION OF THE DRAWINGS

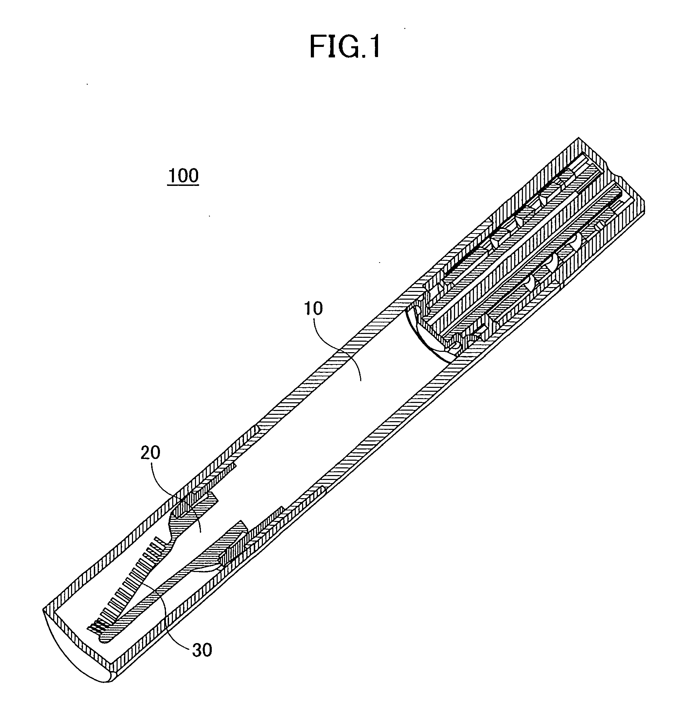

FIG. 1 is a diagram illustrating one example of a container structure of a mascara remover product according to the present invention.

EXPLANATION OF LETTERS OR NUMERALS

    10    mascara remover
    20    ejection port
    30    ejection orifice
    100    feeding container

BEST MODE FOR CARRYING OUT THE INVENTION

The inventors created an idea of a mascara remover product providing uniform adhesion to an eyelash(es), having a moderate hardness to prevent entering into an eye, being easy to apply, being selectively capable of removing a mascara, being economical and involving no risk to a human body.

Furthermore, the inventors created an idea of compounding particular components at particular rates to prepare a mascara remover and puting such a mascara remover in a container suitable for its application to an eyelash(es).

One embodiment of the present invention is a mascara remover product **characterized in that** a mascara remover including (a) an expansive clay mineral and (b) a dextrin fatty acid ester, wherein a total compounding amount of component (a) and component (b) is 5 - 25 % by mass and a compounding ratio of component (a) to component (b) is (a):(b) = (1:5) - (5:1) in a mass ratio, is contained in a feeding container.

A hardness of a mascara remover to be used in a mascara remover product according to an embodiment of the present invention is preferably 5 - 70 when being measured by the following method.

(Method of measuring hardness)

A card tension meter (produced by IIO Electric Co., Ltd.) is used whereby a sample having been stored at 30 °C is set on a movable base plate and raised at a constant speed and a motion of a pressure-sensitive spindle linked with a precision spring on a top thereof is recorded to measure a physical property (hardness, $dyne/cm^2$) possessed by a solidification-like product. For a measurement, measuring is conducted by using a plunger with 8 mmφ and a load of 200 g.

According to one embodiment of the present invention, it may be possible to provide a mascara remover product providing uniform adhesion to an eyelash(es), being easy to apply, being capable of selectively removing a mascara, being economical and involving no risk to an human body.

Furthermore, a mascara remover product according to an embodiment of the present invention is not difficult to eject its content at a low temperature and does not cause liquid dripping even at high temperature, whereby a stable quality may be maintained in and out of season.

Next, an embodiment of the present invention will be described with reference to the accompanying drawings.

In a mascara remover product according to an embodiment of the present invention, an oil gel formed of an expansive clay mineral and an oil gel formed of a dextrin fatty acid ester are compounded at a good balance to provide a mascara remover which adheres to an eyelash(es) uniformly and has a hardness to prevent dripping and such a mascara remover is contained in a feeding-type container which enables application of a constant uniform amount thereof.

((a) Expansive clay mineral)

For an expansive clay mineral that is component (a) in an embodiment of the present invention, for example, a layered silicate (salt) mineral classified as a smectite group is provided in which there is generally provided, for example, a hectorite, a montmorillonite, a beidellite, a nontronite, a saponite, or the like, and these may be a natural or synthetic product. For a commercially available product, for example, there is provided Laponite (a hectorite: Laporte Industries Ltd.), Kunipia (a montmorillonite: Kunimine Industries Co., Ltd.), Sumecton (a saponite: Kunimine Industries Co., Ltd.), Veegum (a bentonite: R. T. Vanderbilt Company, Inc.), a synthetic mica such as a fluorine tetrasilicate mica (for example, commercial name: Dimonite, Topy Industries Inc.), or the like.

These expansive clay minerals are modified by a modifying agent as described below to be used in an embodiment of the present invention, however, even when it is not modified, implementation thereof is also possible. If an expansive clay mineral is modified, the expansive clay mineral described above and a modifying agent as described below are compounded to conduct modification of the expansive clay mineral, for example, when an oil phase of a mascara remover according to an embodiment of the present invention is prepared. Additionally, if a non-expansive clay mineral is to be used, for example, the expansive clay mineral is compounded in an aqueous phase.

For a modifying agent for modifying an expansive clay mineral, for example, a quaternary ammonium salt-type cationic surfactant is used and represented by the following general formula (1):

$$\left( \begin{array}{c} R^2 \\ | \\ R^1 - N - R^3 \\ | \\ R^4 \end{array} \right)^{+} \quad X^{-} \quad \cdots (1)$$

(in which formula, $R^1$ represents an alkyl group with a carbon number of 10 - 22 or a benzyl group, $R^2$ represents a methyl group or an alkyl group with a carbon number of 10 - 22, each of $R^3$ and $R^4$ represents an alkyl group with a carbon number of 1 - 3 or a hydroxyalkyl group, and X represents a halogen atom or a methylsulfate residue.).

If specific ones are provided, it is possible to provide, for example, dodecyltrimethylammonium chloride, myristyltrimethylammonioum chloride, cetyltrimethylammonium chloride, stearyltrimethylammonium chloride, behenyltrimethylammonium chloride,

myristyldimethylethylammonioum chloride,
cetyldimethylethylammonioum chloride,
arachyltrimethylammonium chloride,
stearyldimethylethylammonioum chloride,
arachyldimethylethylammonioum chloride,
behenyldimethylethylammonioum chloride,
myristyldiethylmethylammonioum chloride,
cetyldiethylmethylammonioum chloride,
stearyldiethylmethylammonioum chloride,
arachyldiethylmethylammonioum chloride,
behenyldiethylmethylammonioum chloride,
benzyldimethylmyristylammonioum chloride,
benzyldimethylcetylammonioum chloride,
benzyldimethylstearylammonioum chloride,
benzyldimethylbehenylammonioum chloride,
benzylmethylethylcetylammonioum chloride,
benzylmethylethylstearylammonioum chloride,
distearyldimethylammonioum chloride,
dibehenyldi(hydroxyethyl)ammonium chloride, and
corresponding bromides, and the like, and further dipalmitylpropylethylammonium methylsulfate, and the like.
For an embodiment of the present invention, one kind or two or more kinds of them is/are selected arbitrarily.

A content of a quaternary ammonium salt-type cationic surfactant is preferably 40 - 120 milli-equivalent to 100 g of an expansive clay mineral. Such a surfactant chemically and rigidly bonds to an expansive clay mineral, and may all be consumed in order to modify an expansive clay mineral when the content is as described above, whereby its release from an expansive clay mineral may not be caused after modification thereof.

Furthermore, for a commercial product existing as an originally modified clay mineral, there is provided, for example, Bentone 38VCG (distearyldiammoniumhectorite, produced by Elementis Specialities Inc.) or Bentone 38 (Quaternium-18 hectorite, produced by Elementis Specialities Inc.).

A compounding amount of such an expansive clay mineral is preferably 1 - 20 % by mass, more preferably 3 - 15 % by mass, and most preferably 5 - 10 % by mass, with respect to the total amount. If such a compounding amount is more than 20 % by mass, uniform dissolution may not be caused to produce a dissolution residue or precipitate whereby a difficulty in its stability and usability as a base material may be caused.


((b) Dextrin fatty acid ester)

For a dextrin fatty acid ester that is component (b) used in an embodiment of the present invention, for example, an ester of dextrin and a higher fatty acid with a carbon number of 12 - 22 is used, and specifically, it is possible to provide, for example, a dextrin palmitate, a dextrin (palmitate/2-ethylhexanoate), a dextrin laurate, a dextrin myristate, a dextrin stearate, a dextrin behenate, a dextrin coconut oil fatty acid ester, or the like. Among these, a dextrin palmitate or a dextrin (palmitate/2-ethylhexanoate) is most preferable from the viewpoint of the stability and usability thereof.

For a dextrin fatty acid ester, for example, a dextrin palmitate and a dextrin (palmitate/2-ethylhexanoate) are commercially available as "Rheopearl KL" (produced by Chiba Flour Milling Co., Ltd.) and "Rheopearl TT" (produced by Chiba Flour Milling Co., Ltd.), respectively.

A compounding amount of a dextrin fatty acid ester in an embodiment of the present invention is preferably 1 - 20 % by mass, more preferably 3 - 15 % by mass, and most preferably 5 - 10 % by mass, with respect to the total amount of a mascara remover. If such a compounding amount is less than 1 % by mass, there may be a problem in a temporal stability, and on the other hand, if it is more than 29 % by mass, a content hardness may be so high that it may be difficult to uniformly eject from a feeding container.

In an embodiment of the present invention, a compounding mass ratio of a dextrin fatty acid ester/an expansive clay mineral in a mascara remover is preferably dextrin fatty acid ester/expansive clay mineral = 0.2 - 5, and more preferably 0.3 - 3. If deviating from such a compounding mass ratio, a stability of emulsification may be deteriorated.

Furthermore, in an embodiment of the present invention, a total compounding amount of a dextrin fatty acid ester and an expansive clay mineral in a mascara remover is preferably 5 - 25 % by mass and more preferably 10 - 20 % by mass. If the total compounding amount is less than 5 % by mass, it may be difficult to maintain a content stability due to exudation of an oil component or the like, and if the total compounding amount is more than 25 % by mass, a content hardness may be so high that it may be difficult to uniformly eject from a feeding container.

(Oil component)

In an embodiment of the present invention, an oil component to be used in a mascara remover is composed of one kind or two or more kinds of an oily component(s) and a liquid-like one as a single component or as a solution in another oily component at an ordinary temperature is used, wherein, specifically, it is possible to provide, for example, the following compound.

That is, for a liquid fat and oil, there is provided, for example, a linseed oil, a camellia oil, a macadamia nut oil, a corn oil, a mink oil, an olive oil, an avocado oil, a sasanqua oil, a castor oil, a safflower oil, an apricot kernel oil, a cinnamon oil, a jojoba oil, a grape oil, a sunflower oil, an almond oil, a rapeseed oil, a sesame oil, a wheat germ oil, a rice germ oil, a rice bran oil, a cotton seed oil, a soy bean oil, a peanut oil, tea seed oil, an evening primrose oil, an egg-yolk oil, a liver oil, a coconut oil, a palm oil, a palm kernel oil, or the like.

For a solid fat and oil, there is provided, for example, a cacao butter, a beef tallow, a neat's foot fat, a mutton tallow, a lard, a horse fat, a hardened oil, a hardened castor oil, a shea butter, or the like.

For a wax, there is provide, for example, a bees wax, a candelilla wax, a Japan wax, a cotton wax, a carnauba wax, a bayberry wax, a Chinese insect wax, a spermaceti wax, a montan wax, a bran wax, a lanolin, a reduced lanolin, a hard lanolin, a kapok lanolin, a sugarcane wax, a jojoba wax, a shellac wax, or the like.

For an ester oil, there is provided, for example, an octanoic acid ester such as cetyl octanoate, a lauric acid ester such as hexyl laurate, a myristic acid ester such as isopropyl myristate or octyldodecyl myristate, a palmitic acid ester such as octyl palmitate, a stearic acid ester such as isocetyl stearate, an isostearic acid ester such as isopropyl isostearate, an isopalmitic acid ester such as octyl isopalmitate, an oleic acid ester such as isodecyl oleate, an adipic acid ester such as isopropyl adipate, a sebacic acid ester such as ethyl sebacate, a malic acid ester such as isostearyl malate, glycerin trioctanoate, glycerin triisopalmitate, or the like.

For a hydrocarbon oil, there is provided, for example, a liquid paraffin, an ozokerite, a squalane, squalene, a pristine, a paraffin, an isoparaffin, a light liquid paraffin, a ceresin, Vaseline, a microcrystalline wax, or the like.

For a silicone oil, there is provided, for example, dimethylpolysiloxane, methylphenylpolysiloxane, methylhydrogenpolysiloxane, or the like.

In an embodiment of the present invention, 30 - 80 % by mass of an oil as described above is preferable with respect to the total amount of a mascara remover. If more than 80 % by mass of an oil component is provided, it may be difficult to maintain hardness as a base material. On the other hand, if less than 30 % by mass of an oil component is provided, it may be difficult to obtain a sufficient removal effect.

Furthermore, it is preferable to compound a volatile oil component in order to obtain a sufficient effect of removing a mascara, and in particular, it is more preferable to compound 1 % by mass or more of a light liquid paraffin.

(Aqueous phase)

In an embodiment of the present invention, a compounding amount of an aqueous phase component is preferably 0.5 - 30 % by mass with respect to the total amount. For an aqueous phase component, it is possible to provide, for example, a humectant as described below, in addition to water. If more than 30 % by mass of an aqueous phase component is compounded, an emulsion stability may be deteriorated, which is not preferable.

(Others)

In an embodiment of the present invention, emulsification is conducted by using a surfactant so that it may be possible to prepare a mascara remover.

For a lipophilic and non-ionic surfactant in a usable surfactant, it is possible to present, for example, a sorbitan fatty acid ester such as sorbitan monooleate, sorbitan monoisostearate, sorbitan monolaurate, sorbitan monopalmitate, sorbitan monostearate, sorbitan sesuquioleate, sorbitan trioleate, diglycerol sorbitan penta-2-ethylhexylate, or diglycerol sorbitan tetra-2-ethylhexylate; a glycerin fatty acid ester such as a glycerin cotton seed oil fatty acid ester, glyceryl monoerucate, glyceryl sesquioleate, glyceryl monostearate, or $\alpha,\alpha'$-glyceryl oleate pyroglutamate; a propylene glycol fatty acid ester such as propylene glycol monostearate; a hardened castor oil derivative; an alkyl glyceryl ether; or the like.

Furthermore, for a hydrophilic and non-ionic surfactant, it is possible to provide, for example, a polyoxyethylene sorbitan fatty acid ester, a polyoxyethylene sorbitol fatty acid ester, a polyoxyethylene glycerin fatty acid ester, a polyoxyethylene fatty acid ester, a polyoxyethylene alkyl ether, a polyoxyethylene alkylphenyl ether, a polyoxyethylene-polyoxypropylene block copolymer, a polyoxyethylenepolyoxypropylene alkyl ether, a tetrapolyoxyethylene or tetrapolyoxypropylene derivative of ethylenediamine, a polyoxyethylene castor oil derivative, a polyoxyethylene hardened castor oil derivative, a polyoxyethylene derivative of a bees wax or lanolin, an alkanolamide, a polyoxyethylenepropylene glycol fatty acid ester, a polyoxyethylenealkylamine, a polyoxyethylene fatty amide, a sucrose fatty acid ester, a polyoxyethylene and nonylphenyl derivative of formaldehyde, an alkylethoxydimethylamine oxide, trioleyl phosphate, or the like.

For an anionic surfactant, it is possible to provide, for example, a fatty acid soap, a higher alkyl sulfate (ester salt), an alkyl ether sulfate (ester salt), an N-acylsarcosinic acid, a higher fatty amide sulfonate, a phosphate (ester salt), a sulfosuccinate (salt), an alkylbenzenesulfonate (salt), a higher fatty acid ester sulfate (ester salt), a sulfonated oil, a polyoxyethylene alkyl ether carboxylic acid, a polyoxyethylene alkylallyl ether carboxylate (salt), an $\alpha$-olefinsufonate (salt), a higher fatty acid ester sulfonate (salt), a secondary alcohol sulfate (ester salt), a higher fatty acid alkylolamide sulfate (ester salt), sodium lauroylmonoethanol amide succinate, N-palmitoylaspartic acid ditriethanolamine, a sodium casein, or the like.

For a cationic surfactant, it is possible to provide, for example, an alkyltrimethylammonium salt, an alkylpyridinium salt, an alkyl(quaternary)ammonium salt, an alkyldimethylbennzylammoniumm salt, an alkylisoquinolinium salt, a dialkylmorpholinium salt, an alkylamine salt, a fatty acid derivative of alcohol, a benzalkonium chloride, benzethonium chloride, or the like.

For an amphoteric surfactant, it is possible to provide, for example, an imidazoline-type amphoteric surfactant, a betaine-type surfactant, or the like.

It may be possible to appropriately determine an amount of a surfactant to be used while a feature and content of an oily component are taken into consideration.

Among these surfactants, it is particularly preferable to use a polyoxyethylene-methylpolysiloxane copolymer and a triisostearic acid ester of polyoxyethylene hardened castor oil in combination in an embodiment of the present invention from the viewpoint of a temperature stability.

Among these, for a polyoxyethylene-methylpolysiloxane copolymer, it is possible to provide, for example, a PEG-11 methyl ether dimeticon ("KF-6011"; produced by Shin-Etsu Chemical Co., Ltd.), a PEG-9 dimeticon ("KF-6013"; produced by Shin-Etsu Chemical Co., Ltd.), a PEG-3 dimeticon ("KF-6015"; produced by Shin-Etsu Chemical Co., Ltd.), a PEG-9 methyl ether dimeticon ("KF-6016"; produced by Shin-Etsu Chemical Co., Ltd.), a PEG-10 dimeticon ("KF-6017"; produced by Shin-Etsu Chemical Co., Ltd.), a PEG-11 methyl ether dimeticon ("KF-6018"; produced by Shin-Etsu Chemical Co., Ltd.), a PEG-9 dimeticon ("KF-6019"; produced by Shin-Etsu Chemical Co., Ltd.), a PEG-12 dimeticon ("SH3771M", "SH3772M", "SH3773M", "SH3775M" or the like, produced by Dow Corning Toray Co.,Ltd.), or the like.

For a triisostearic acid ester of polyoxyethylene hardened castor oil, it is possible to provide, for example, EMALEX RWIS-310 (produced by Nihon Emulsion Co., Ltd.).

In an embodiment of the present invention, it is possible to compound an arbitrary component other than those described above, in a qualitative or quantitative range which does not impair the effect of an embodiment of the present invention, and it may be possible to compound a component that is commonly compounded into a cosmetic material, for example, a humectant, a thickening agent or dispersion stabilizer, a chelating agent, an antioxidant, an antiseptic agent, a perfume, each kind of vitamin, a coloring agent, an ultraviolet absorber, a drug component, an inorganic salt, or the like.

For a humectant, it is possible to provide, for example, a polyethylene glycol, propylene glycol, glycerin, 1,3-butylene glycol, xylitol, sorbitol, maltitol, a chondroitin sulfate, hyaluronic acid, mucoitin sulfuric acid, karonin acid, an atelocollagen, cholesteryl-12-hydroxystearate, sodium lactate, a bile salt, dl-pyrolidonecarboxylate (salt), a short-chain soluble collagen, a propylene oxide (or an ethylene oxide) adduct of diglycerin, or another extract from a natural living organism, or the like.

For a water-soluble polymer useful for a thickening agent or dispersion stabilizer, it is possible to provide, for example, a natural water-soluble polymer including a polymer of plant origin such as a gum arabic, a tragacanth gum, a galactan, a guar gum, a carob gum, a karaya gum, a carageenan, a pectin, an agar, a starch, or a glycyrrhizic acid, a polymer of microorganism origin such as a xanthan gum, a dextran, a succinoglucan, or a pullulan, or a polymer of animal origin such as a collagen, a casein, an albumin, or a gelatin; a semisynthetic water-soluble polymer such as a starch-type polymer, a cellulose-type polymer, or an alginic acid-type polymer; a copolymerization-type polymer such as a vinyl-type polymer, a polyoxyethylene-type polymer, or a polyoxyethylene-polyoxypropylene copolymer; or a synthetic water-soluble polymer such as an acryl-type polymer, a polyethyleneimine, or a cationic polymer.

For a chelating agent, it is possible to provide, for example, 1-hydroxyethane-1,1-diphosphonic acid, tetrasodium 1-hydroxyethane-1,1-diphosphonate, disodium edetate, trisodium edetate, tetrasodium edetate, sodium citrate, a sodium polyphosphate, a sodium metaphosphate, gluconic acid, phosphoric acid, citric acid, ascorbic acid, succinic acid, edetic acid, or the like.

A hardness of a mascara remover in an embodiment of the present invention is preferably 5 - 70 (unit: dyne/cm$^2$) and particularly preferably 10 - 50 when being measured by a method as described above.

If the hardness is more than 70, a content hardness may be so high that it may be difficult to attain uniform ejection from a feeding container. On the other hand, if the hardness is less than 5, it may be difficult to maintain a content stability due to exudation of an oil component or the like and it may be difficult to attain its containment in a feeding container and its use due to liquid leakage or the like. Furthermore, it may be difficult to hold liquid on an eyelash (es) so as not to enter an eye at the time of application.

A mascara remover product according to an embodiment of the present invention is such that a feeding container is filled with a mascara remover as described above, and specifically, it is possible to illustrate, for example, a feeding container 100 as illustrated in FIG. 1.

A mascara remover 10 is contained inside such a feeding container 100 and it is possible to push such a mascara remover 10 toward a tip part thereof. A structure is provided such that a mascara remover 10 is ejected from a comb teeth-shaped ejection orifice 30 at the tip of the container through an ejection port 20 provided in the container.

It is possible for such a feeding container 100 to eject a viscous base material uniformly without excessive ejection thereof.

[Examples]

An embodiment of the present invention will be further described in detail from the following practical examples, however, the present invention is not limited to them. Additionally, compounding ratios will be presented in % by mass unless otherwise particularly stated.

[Practical examples 1 - 6 and comparative examples 1 and 2 (mascara remover)]

A mascara remover with a formulation presented in the following Table 1 was prepared by an ordinary method and its hardness (unit: dyne/cm$^2$) was measured.

A feeding container as illustrated in FIG. 1 was filled with the mascara remover to provide a mascara remover product. A content stability, an adhesion to eyelashes, an operability in the feeding container at 5 °C, 20 °C, or 35 °C, or a mascara removing effect for the obtained mascara remover product were measured in the following criteria. Its results were also presented in Table 1.

(1) Content stability

A feeding container was filled with a mascara remover to provide a mascara remover product and presence or absence of exudation of an oil at 0 °C, room temperature, or 37 °C was evaluated after one month.

(Evaluation criteria)

A: There was no exudation of an oil.
B: There was exudation of an oil.

(2) Adhesion to eyelashes

Adhesion to eyelashes was evaluated when application was made to the eyelashes at 20 °C by using a mascara remover product.

(Evaluation criteria)

A: Adhesion was very good.
B: Adhesion was good.
C: Adhesion was not so good.
D: Adhesion was bad.

(3) Operability in a feeding container

Ease of an operation in a feeding container was evaluated at each of temperatures of 5 °C, 20 °C, and 35 °C.

(Evaluation criteria)

A: Operability was very good.
B: Operability was good.
C: Slightly difficult or there was slight exudation of an oil.
D: Difficult or there was exudation of an oil.

(4) Mascara removing effect

Ease of removal of a mascara was evaluated when the mascara was removed by using a mascara remover product.

(Evaluation criteria)

A: Very easy to remove
B: Easy to remove
C: Slightly difficult to remove
D: Difficult to remove

Table 1

| | Practical example 1 | Practical example 2 | Practical example 3 |
|---|---|---|---|
| Light liquid paraffin | – | – | – |
| Liquid paraffin | 42 | 42 | 42 |
| Cetyl 2-ethyhexanoate | 10 | 10 | 10 |
| Neopentyl glycol dicaprate | 10 | 10 | 10 |
| Methylpolysiloxane | 10 | 10 | 10 |
| Dextrin palmitate | 7.5 | 10 | – |
| Dextrin (palmitate/2-ethylhexanoate) | – | 5 | 5 |
| Distearyl diammonium hectorite *1 | 7.5 | 5 | 15 |
| Polyoxyethylene-methylpolysiloxane copolymer *2 | 2 | 2 | 2 |
| Triisostearic acid polyoxyethylene hardened cator oil | 2 | 2 | 2 |
| 1,3-butyrene glycol | 3 | 3 | 3 |
| Purified water | 1 | 1 | 1 |
| Total | 100 | 100 | 100 |
| Hardness | 30 | 65 | 25 |
| – Content stability (presence or absence of exudation of oil at time passage of 1 M) | A | A | A |
| – Adhesion to eyelashes (application from feeding container at 20 °C) | A | B | B |
| – Operability for feeding container    5 °C | A | B | A |
| 20 °C | A | A | A |
| 35 °C | A | A | B |
| – Mascara removing effect | B | B | B |

Table 1 – Continued.

| | Practical example 4 | Practical example 5 | Practical example 6 |
|---|---|---|---|
| Light liquid paraffin | 10 | 20 | – |
| Liquid paraffin | 37 | 27 | 52 |
| Cetyl 2-ethyhexanoate | 10 | 10 | 10 |
| Neopentyl glycol dicaprate | 10 | 10 | 10 |

9

| | | | |
|---|---|---|---|
| Methylpolysiloxane | 10 | 10 | 10 |
| Dextrin palmitate | 7.5 | 7.5 | 5 |
| Dextrin (palmitate/2-ethylhexanoate) | – | – | – |
| Distearyl diammonium hectorite *1 | 7.5 | 7.5 | 5 |
| Polyoxyethylene-methylpolysiloxane copolymer *2 | 2 | 2 | 2 |
| Triisostearic acid polyoxyethylene hardened cator oil | 2 | 2 | 2 |
| 1,3-butyrene glycol | 3 | 3 | 3 |
| Purified water | 1 | 1 | 1 |
| Total | 100 | 100 | 100 |
| Hardness | 40 | 50 | 15 |
| - Content stability (presence or absence of exudation of oil at time passage of 1 M) | A | A | A |
| - Adhesion to eyelashes (application from feeding container at 20 °C) | A | A | B |
| - Operability for feeding container    5 °C | A | A | A |
| 20 °C | A | A | A |
| 35 °C | A | A | A |
| - Mascara removing effect | A | A | B |

Table 1 – Continued.

| | Comparative example 1 | Comparative example 2 |
|---|---|---|
| Light liquid paraffin | – | – |
| Liquid paraffin | 42 | 42 |
| Cetyl 2-ethyhexanoate | 10 | 10 |
| Neopentyl glycol dicaprate | 10 | 10 |
| Methylpolysiloxane | 10 | 10 |
| Dextrin palmitate | 20 | – |
| Dextrin (palmitate/2-ethylhexanoate) | – | – |
| Distearyl diammonium hectorite *1 | – | 20 |
| Polyoxyethylene- | 2 | 2 |

| | | |
|---|---|---|
| methylpolysiloxane copolymer *2 | | |
| Triisostearic acid polyoxyethylene hardened cator oil | 2 | 2 |
| 1,3-butyrene glycol | 3 | 3 |
| Purified water | 1 | 1 |
| Total | 100 | 100 |
| Hardness | 120 | 30 |
| - Content stability (presence or absence of exudation of oil at time passage of 1 M) | A | B |
| - Adhesion to eyelashes (application from feeding container at 20 °C) | C | C |
| - Operability for feeding container    5 °C | D (Difficult) | B |
| 20 °C | D (Difficult) | B |
| 35 °C | B | D (oil exudation) |
| - Mascara removing effect | C | B |

* 1: Bentone 38 VCG (produced by Elementis Specialities Inc.)

* 2: Silicone KF-6017 (produced by Shin-Etsu Chemical Co., Ltd.)

[Practical example 7]

Light liquid paraffin        10 % by mass
Liquid paraffin         20 % by mass
Cetyl 2-ethyhexanoate         15 % by mass
Neopentyl glycol dicaprate        15 % by mass
Methylpolysiloxane        5 % by mass
Dextrin palmitate        7 % by mass
Dextrin (palmitate/2-ethylhexanoate)        3 % by mass
Distearyl diammonium hectorite *1        5 % by mass
Polyoxyethylene-methylpolysiloxane copolymer *2        4 % by mass
Triisostearic acid polyoxyethylene hardened cator oil        1 % by mass
1,3-butyrene glycol        5 % by mass
Purified water        10 % by mass

11

[Practical example 8]

| | |
|---|---|
| Light liquid paraffin | 10 % by mass |
| Liquid paraffin | 25 % by mass |
| Cetyl 2-ethyhexanoate | 10 % by mass |
| Neopentyl glycol dicaprate | 10 % by mass |
| Methylpolysiloxane | 10 % by mass |
| Dextrin palmitate | 5 % by mass |
| Distearyl diammonium hectorite *1 | 5 % by mass |
| Polyoxyethylene-methylpolysiloxane copolymer *2 | 3 % by mass |
| Triisostearic acid polyoxyethylene hardened cator oil | 2 % by mass |
| 1,3-butyrene glycol | 3 % by mass |
| Purified water | 7 % by mass |

[Appendix]

Appendix (1): A mascara remover product **characterized in that** a mascara remover including (a) an expansive clay mineral and (b) a dextrin fatty acid ester, wherein a total compounding amount of component (a) and component (b) is 5 - 25 % by mass and a compounding ratio of component (a) to component (b) is (a):(b) = (1:5) - (5:1) in mass ratio, is contained in a feeding container.

Appendix (2): The mascara remover product as described in appendix (1) **characterized in that** a hardness of the mascara remover when being measured by the following method is 5 - 70.

(Method for measuring hardness)

A card tension meter (produced by IIO Electric Co., Ltd.) is used whereby a sample having been stored at 30 °C is set on a movable base plate and raised at a constant speed and a motion of a pressure-sensitive spindle linked with a precision spring on a top thereof is recorded to measure a physical property (hardness, $dyne/cm^2$) possessed by a solidification-like product. For a measurement, measuring is conducted by using a plunger with 8 mm$\phi$ and a load of 200 g.

Appendix (3): The mascara remover product as described in appendix (1) **characterized in that** the mascara remover is a water-in-oil-type emulsion.

Appendix (4): A cosmetic material removing agent, including an expansive clay mineral and a dextrin fatty acid ester.

Appendix (5): The cosmetic material removing agent as described in appendix (4), wherein a total content of the expansive clay mineral and the dextrin fatty acid ester is 5 - 25 % by mass.

Appendix (6): The cosmetic material removing agent as described in appendix (4) or (5), wherein a ratio of a content of the dextrin fatty acid ester to a ratio of the expansive clay mineral is 0.2 or more and 5 or less.

Appendix (7): The cosmetic material removing agent as described in any one of appendices (4) to (6), wherein a hardness of the cosmetic material removing agent is 5 or more and 70 or less.

Appendix (8): The cosmetic material removing agent as described in any one of appendices (4) to (7), wherein the cosmetic material removing agent is a water-in-oil-type emulsion.

Appendix (9): The cosmetic material removing agent as described in any one of appendices (4) to (8), wherein the cosmetic material removing agent is a mascara remover.

Appendix (10): A cosmetic material removing device including the cosmetic material removing agent as described in any one of appendices (4) to (9) and a container for containing and feeding the cosmetic material removing agent.

Although an embodiment(s) and an example(s) of the present invention have been specifically described above, the present invention is not limited to the embodiment(s) or example(s) and it is possible to alter or modify the embodiment (s) or example(s) of the present invention without departing from the scope of the present invention.

INDUSTRIAL APPLICABILITY

One embodiment of the present invention may be available for a mascara remover product. For example, one embodiment of the present invention may be preferable for being contained in a feeding container and used and may be available for a mascara remover product with a good usability.

The present application claims the benefit of its priority based on Japanese Patent Application No. 2008-000616 filed on January 7, 2008 in Japan, the entire content of which is hereby incorporated by reference.

**Claims**

1. A cosmetic material removing agent comprising an expansive clay mineral and a dextrin fatty acid ester.

2. The cosmetic material removing agent as claimed in claim 1, wherein a total content of the expansive clay mineral and the dextrin fatty acid ester is 5 - 25 % by mass.

3. The cosmetic material removing agent as claimed in claim 1, wherein a ratio of a content of the dextrin fatty acid ester to a content of the expansive clay mineral is 0.2 or more and 5 or less.

4. The cosmetic material removing agent as claimed in claim 1, wherein a hardness of the cosmetic material removing agent is 5 or more and 70 or less.

5. The cosmetic material removing agent as claimed in claim 1, wherein the cosmetic material removing agent is a water-in-oil-type emulsion.

6. The cosmetic material removing agent as claimed in claim 1, wherein the cosmetic material removing agent is a mascara remover.

7. A cosmetic material removing device comprising the cosmetic material removing agent as claimed in claim 1 and a container for containing and feeding the cosmetic material removing agent.

8. A cosmetic material removing device comprising the cosmetic material removing agent as claimed in claim 2 and a container for containing and feeding the cosmetic material removing agent.

9. A cosmetic material removing device comprising the cosmetic material removing agent as claimed in claim 3 and a container for containing and feeding the cosmetic material removing agent.

10. A cosmetic material removing device comprising the cosmetic material removing agent as claimed in claim 4 and a container for containing and feeding the cosmetic material removing agent.

11. A cosmetic material removing device comprising the cosmetic material removing agent as claimed in claim 5 and a container for containing and feeding the cosmetic material removing agent.

12. A cosmetic material removing device comprising the cosmetic material removing agent as claimed in claim 6 and a container for containing and feeding the cosmetic material removing agent.

# FIG.1

100

10

20

30

## INTERNATIONAL SEARCH REPORT

| International application No. |
| --- |
| PCT/JP2008/072417 |

**A. CLASSIFICATION OF SUBJECT MATTER**
*A61K8/25*(2006.01)i, *A45D34/04*(2006.01)i, *A61K8/06*(2006.01)i, *A61K8/73*
(2006.01)i, *A61Q1/14*(2006.01)i

According to International Patent Classification (IPC) or to both national classification and IPC

**B. FIELDS SEARCHED**

Minimum documentation searched (classification system followed by classification symbols)
A61K8/25, A45D34/04, A61K8/06, A61K8/73, A61Q1/14

Documentation searched other than minimum documentation to the extent that such documents are included in the fields searched
Jitsuyo Shinan Koho                  1922-1996   Jitsuyo Shinan Toroku Koho   1996-2009
Kokai Jitsuyo Shinan Koho     1971-2009   Toroku Jitsuyo Shinan Koho   1994-2009

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 3332917 B2  (Noevir Co., Ltd.), | 1-4,6-10,12 |
| Y | 26 July, 2002 (26.07.02),<br>Claims; Par. Nos. [0009], [0021], [0035],<br>[0037]; Figs. 1, 2; examples<br>& JP 2003-104839 A | 1-4,6-10,12 |
| Y | JP 3-115207 A  (Kabushiki Kaisha Kobayashi Kose),<br>16 May, 1991 (16.05.91),<br>Page 2, upper left column, lines 2 to 8;<br>page 2, lower right column, line 15 to<br>page 3, upper left column, line 11<br>(Family: none) | 1-4,6-10,12 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 March, 2009 (06.03.09) | 17 March, 2009 (17.03.09) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/072417

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2001-200160 A  (Noevir Co., Ltd.),<br>24 July, 2001 (24.07.01),<br>Par. No. [0002]<br>(Family: none) | 1-4,6-10,12 |
| Y | JP 2002-179548 A  (Shin-Etsu Chemical Co., Ltd.),<br>26 June, 2002 (26.06.02),<br>Par. No. [0058]; example 20<br>& EP 1213011 A1        & US 6790451 B2 | 1-4,6-10,12 |
| P,A | JP 2008-247785 A  (Naris Cosmetics Co., Ltd.),<br>16 October, 2008 (16.10.08),<br>Claims<br>(Family: none) | 1-12 |
| P,A | JP 2008-247846 A  (Kose Corp.),<br>16 October, 2008 (16.10.08),<br>Claims<br>(Family: none) | 1-12 |
| P,A | JP 2008-44868 A  (Shiseido Co., Ltd.),<br>28 February, 2008 (28.02.08),<br>Claims<br>(Family: none) | 1-12 |
| A | JP 9-002816 A  (Kose Corp.),<br>07 January, 1997 (07.01.97),<br>Par. No. [0007]; example 6<br>(Family: none) | 1-12 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003104839 A
- JP 2008000616 A